# EUROPEAN PATENT APPLICATION

(11) **EP 4 596 764 A1**
(43) Date of publication of application: **06.08.2025**
(21) Application number: 24161946.9
(22) Date of filing: 07.03.2024
(51) Int. Cl.: D01D 5/098, D01D 7/00, D01D 13/02, D04H 3/02, D04H 3/14, A61F 13/15

(54) **INTERMITTENT APPLICATION OF WEBS WITH CONTINUOUS FILAMENTS**

(30) Priority: 01.02.2024 EP 24155366
(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is an apparatus and a process for forming a fibrous web comprising continuous filaments, whereby the equipment respectively the process is adapted to be connected to further converting units respectively processing steps in a manufacturing process, whereby at an interruption of any of the further processing steps the filaments made during the interruption are captured for being reused by diverting the produced filaments.

## Description

### Field of the invention

The present invention relates to an apparatus and a process for forming a fibrous web comprising continuous filaments, whereby the apparatus respectively the process is adapted to be connected to further converting units respectively processing steps in a manufacturing process for absorbent articles, whereby at an interruption of any of the further processing steps the filaments made during the interruption are captured for being reused by diverting the produced filaments.

### Background

Manufacturing of continuous filaments from thermoplastic polymers is well known, in particular for the formation of fibrous webs.

Such filaments may be formed by melting polymer in an extruder and pressing the molten polymer through nozzles. Thereafter, the filaments are cooled, more or less attenuated and deposited onto a collection device, as may be foraminous belts, drums or combinations thereof. When reaching the collection surface, the filaments typically exhibit some stickiness due to the increased temperature resulting in some consolidation of the web.

One area for using webs comprising continuous filaments is the hygiene industry, be it for backsheet, topsheets or absorbent structures, with all applications being well known in the art for forming hygiene articles, such as diapers. Such articles are produced on a "converter", a machine for combining multiple webs and bulk materials.

A first approach for such filament forming is known as spunbonding, whereby filaments of molten polymer are expelled in a spinnerette with a multitude of orifices, quenched, and then strongly attenuated to provide essentially continuous filaments of about 1 to 50 µm, often between 15 µm and 50 µm. The strong attenuation realigns the orientation of the polymer chains in the filament, thusly increasing the strength of the filaments. Typical basis weight of the resulting webs are often more than about 13 g/m² though lower basis weights are aimed for in modern equipment. The spinnerets may have production widths of more than 1 m, often more than 3 m, or even more than 5 m.

Another approach for creating filaments uses meltblowing technology, whereby the filaments expelled from nozzles can be much thinner and are less attenuated.

Within the meltblowing technology, the formation of continuous filaments needs to be distinguished from forming discontinuous fiber webs, the latter also referred to as "convergent melt-blowing processes" with forming of fibrous webs according to the teachings of e.g., US3849241 (Butin, K-C), whereby molten polymer is formed into filaments and treated with hot air streams in an angled configuration, i.e. between the filaments path after the nozzle and hot quenching air direction there is an angle of typically between 30° and 60°. Thereby, the filaments are separated into individual non-continuous fibers which are collected to form a web.

More recently, forming of continuous meltblown filaments was described by e.g., Biax Fiberfilm Corp. referred to as Spun-blowing ^{®} or coaxial meltblowing, whereby each nozzle of an array of nozzles is surrounded by a hot annular air flow, not disrupting the filaments whilst cooling and attenuating these, though to a lesser degree than for the spunbonding technology. Such a technology is also referred to a coaxial meltblowing, as may be abbreviated CAM or CoAx, known from e.g., EP3140447 (Schwarz) or WO2021/089731 (TKWM). In this approach, the filaments exhibit a diameter that may be less than 1 µm and basis weight of less than about 1 g/m², with larger diameter of up to about 20 µm and higher basis weights of more than about 50 g/m² can be quite readily achieved.

It is also known to add particulates to webs, in particular to webs of continuous filaments. In case of adding short cellulosic fibers, it is referred to as "co-forming", see e.g., US4100324. The particulate material may be absorbent particles for absorbing liquid, with gel forming or so-called superabsorbent materials, as well-known in the art of absorbent hygiene articles, see e.g., above mentioned WO2021/089731 (TKWM). In such an approach the meltblown fibers may also be formed from a propylene/α-olefin copolymer, see e.g., US20090233019A1, whereby the more elastic properties of the polymer are believed to ease expansion of the superabsorbent particles upon swelling.

When webs with continuous filaments are used in such a production, these are preformed, i.e., produced off-line at high efficiency and high production speed on large production units exhibiting large width. However, this approach also induces significant cost, as the material, typically wound up in large "mother rolls" need to be cut to appropriate width and be transported to the converter site. As such webs may be lofty, say low density, materials, significant amount of air is being transported at significant cost in addition to the add-on handling effort. Further, differing sizes of articles require different web widths, which has to be considered logistically, increasing complexity and also cost.

Thus, forming such a web "in-line" on a converter would be desirable, but is so far not practised as the extrusion process cannot be readily stopped, especially when other process steps on the converter go off due to various reasons, such as failures, lack of material supply etc., and the complete converter need to be stopped, which would either create problems in the filament forming process, such as burning and/or degradation of the polymer, or enormous scrap, if the filament feeding towards the line would be maintained.

Henceforth it is an object of the present invention to address the problems of forming webs with continuous filaments in-line with an apparatus that can be integrated into a converter without creating excessive scrap in case of stopping the converter.

### Summary

In a first aspect, the present invention is an apparatus for the production of a web comprising continuous filaments. The apparatus exhibits a machine direction (MD), a cross-machine direction (CD) and a height direction and is adapted to be connected MD directionally upstream and/or downstream to further processing units for forming composite structures comprising the web. The apparatus comprises
a) a frame adapted to connect systems of the apparatus stationarily or movably thereto;
b) a primary filament collecting system connected to the frame directly or indirectly, and comprising at least one primary collection surface adapted to form thereon a web of filaments and transferring the web towards a downstream processing unit;
c) an essentially continuously operating filament forming system comprising
   c1) a polymer supply and extruding unit adapted to melt polymer;
   c2) a molten polymer filament expelling unit being movably connected to the frame so as to allow repositioning relative to the primary collection system;
   c3) a filament quenching and attenuation system;
d) a housing circumscribing a space between the filament forming device and the primary filament collecting device, the housing comprising
   d1) a first opening to receive the filaments from the filament expelling unit and a second opening towards the primary collection unit;
   d2) movable cross-directionally oppositely positioned portions adapted to open the space at least laterally;
e) a control and adjustment system for adapting process conditions of the apparatus, the process conditions comprising at least
   ∘ a polymer temperature at the filament expelling unit,
   ∘ a polymer throughput through the filament expelling unit,
   ∘ a quenching air flow & temperature regulating unit;
   wherein the control and adjustment system is adapted to receive a signal from the further processing unit(s) indicating regular production state or non-production state;
   wherein
e) the apparatus further comprises a secondary filament collection system comprising
   - e1) a distance adjustment system to adjust the distance between the filament expelling device and the primary filament collecting surface to a first production state distance or a second non-production state distance;
   - e2) a cross-directional gas flow system comprising a blower and suction unit for creating a cross-directional air flow between the primary collection surface and the filament expelling unit during the non-production state,
   - e3) a housing relocation system adapted to move the cross-directionally oppositely positioned portions of the housing so as to allowing the cross-directional airflow to contact the filaments and transferring these cross-directionally during the non-production state;
   - e4) a secondary filament transfer system adapted to receive the cross-directionally transferred filaments and to put these into a storage system;
   wherein the secondary filament collection system is operated upon the signal from the control and adjustment system toggling between regular production and non-production state.

The apparatus may further comprise one or more of the features selected from the group consisting of:
- the continuously operating filament forming system is selected from the group consisting of spunbonding and coaxial meltblowing, preferably coaxial meltblowing;
- the primary filament collecting device is selected from the group consisting of
   ∘ a foraminous belt system;
   ∘ a double foraminous drum system;
   ∘ a foraminous belt and drum system;
- the cross-directional gas flow system is adapted to cool the filaments below a polymer stickiness temperature;
- the polymer extruding unit and the filament expelling unit are connected and jointly moved by the distance adjustment system;
- the polymer filament expelling unit is rotatably mounted with an axis parallel to the direction of the expelled filaments;
- the housing opening and relocation system comprises a set of at least two shells positioned cross-directionally opposite and adapted to be at least cross-directionally spaced apart.

Optionally, the apparatus may further comprise a filament recycling system of the filaments from the secondary filament transfer system of the secondary filament collection system, preferably comprising a polymer granulator and a mixer for feeding fresh and recycled polymer to the polymer extruding unit.

The apparatus may even further comprise a particulate supply and feeding system, preferably through further openings of the housing, adapted to feed particulate additives to be intermingled with the filaments during the production state but not during the non-production state.

The apparatus may be positioned downstream of a preformed web supply unit for positioning a preformed web between the primary filament collection unit and web of filaments formed thereon. In another aspect, the present invention is a composite web forming equipment comprising two or more such apparatuses, adapted to toggle between a production state to a non-production state by a common control system receiving a signal from further production units.

In yet another aspect, the present invention is a process for the production of a web of continuous filaments, which is connected machine-directionally (MD) upstream and/or downstream to further processing steps on further processing units for forming composite structures comprising the web. The process is operated along a main machine direction and further exhibiting a cross-direction (CD) and a z- or height direction, rectangularly to MD and CD.

The process comprises the steps of
A) providing such an apparatus or equipment and a polymer for forming filaments;
B) operating the one or more essentially continuously operating continuous filament forming system(s) at regular production process conditions of at least
   ∘ a polymer temperature at the filament expelling unit,
   ∘ a polymer throughput through the filament expelling unit,
   ∘ a quenching air flow & temperature at a quenching air flow & temperature regulating unit;
C) interrupting the step B) upon receipt of a signal from a further processing unit(s), preferably via a central control unit indicating non-production state,
   thereby essentially concurrently
   C1) adjusting the process conditions of the filament forming system according to pre-set non-production process conditions;
   C2) activating the secondary filament collection system by
      C2i) initiating the housing opening and relocation system so as to
         ∘ increase the distance between the filament expelling device and the primary filament collecting surface, and
         ∘ opening movable cross-directionally oppositely positioned portions of the housing so as to open the space at least laterally;
      C2ii) initiating the cross-directional gas flow system so as blow the filaments laterally towards the secondary filament transfer system, thereby preferably reducing the temperature of the filaments below a non-tack temperature;
      C2iii) initiating the secondary filament transfer system, preferably transferring the filaments collected thereon to filament recycling system,
   whereby the steps B) and C) are alternatingly operated upon receipt of the signal from a further processing unit or the central control unit.

Preferably, the transition time between the production state (step B) and the non-production state (step C) is less than 10 sec, preferably less than 5 sec, more preferably less than 1 sec, or more than about 10 msec.

The polymers may be selected from the group consisting of polyolefins, preferably polypropylene, polypropylene copolymers, polyethylene, polyethylene copolymers, polyesters, polyamides, polyimide, polylactic acid, polyhydroxyalkanoate, polyvinyl alcohol, ethylene vinyl alcohol, nylon, polyacrylates, and copolymers thereof and mixtures thereof.

### Brief description of the Figures

Fig. 1 shows a set-up of an equipment and an apparatus according to the present invention in a continuously operating production system.
Fig. 2A and B depict an apparatus according to the present invention in a regular production state and non-production state, respectively.
Fig. 3A to C depict various executions for elements of an apparatus according to the present invention.
Fig. 4 shows an equipment comprising several apparatuses according to the present invention.
The figures are schematic only, and not to scale. Same numerals refer to same or equivalent features or elements, single (`) or multiple (", '", ...) apostrophes indicate duplicate features, such a left and
right or front and back, etc.

### Detailed description

Within the present context, the term "filament" refers to a polymeric filament, whereby at least one polymeric material is provided from a polymer supply system, where polymer, e.g., in the form of pellets or granules, is molten in an extruder and fed to at least one die head as a polymeric filament expelling unit. Aided by vacuum, e.g., of a suction box, positioned under the lower outlet of a housing or forming box, the filaments are laid onto a collection system, such as a moving screen, or a single or two counterrotating foraminous vacuum drum(s).

In case of spunbonding, the filaments are quenched and subsequently attenuated aerodynamically by realigning the polymeric chains, thus increasing the strength of the fibers.

In the preferred case of using the coaxial filament forming technology, each filament expelling nozzle is circumscribed by an annulus through which attenuation air is blown, essentially parallel to the direction into which the filaments are expelled. Such process is well known, such as from EP3140447 (Schwarz) or WO2021/089731 (TKWM). This may also be referred to as coaxial or CoAx meltblowing, and has to be seen in contrast to angular meltblowing, where the attenuation air is expelled angularly to the filament and the filament is separated into discrete fibers, such as described in US3849241 (Butin, K-C). The CoAx die head comprises an array of at least 2 rows, or more than 4 rows, or more than 8 rows of orifices through which molten polymer is expelled, each orifice being circumscribed by a concentric annulus delivering compressed air. In the array, the orifices are arranged in columns, perpendicularly arranged to the rows and may comprise in large production units thousands of nozzles. An exemplary execution of such an apparatus as well as its operation is described in more detail and with multiple variants, e.g., in WO2021089731A1 (TKWM), to which express reference is made for the forming of the filaments.

The polymeric material for forming the filaments may be any thermoplastic extrudable polymer being - in contrast to adhesives such as hot melts - essentially non-tacky at normal use conditions, respectively below a non-tack temperature corresponding to the point where at least 50%, or more than about 80% or more than about 95%, or even all of the filaments do not adhere to another filament, as can be establish by tack tests, such a well-established for adhesives. Thus, suitable materials may be without limitation polyolefins (polypropylene and polypropylene copolymers, polyethylene and polyethylene copolymers), polyesters, polyamides, polyimide, polylactic acid, polyhydroxyalkanoate, polyvinyl alcohol, ethylene vinyl alcohol, nylon, polyacrylates, and copolymers thereof and mixtures thereof. The filaments may further comprise additives, such as hydrophilicity adjustment agents such as nano-cellulose, or an opacifying agent. For certain applications, such as when the filaments are to be intermingled with particulate material such as superabsorbent particles, more stretchable polymers may be preferred, such as thermoplastic elastomers, such as by addition of propylene/α-olefin copolymers to polypropylene, as known from e.g., US2009/0233049.

The term "substrate" or "web" is used herein to describe a material which is primarily two-dimensional (i.e., in cartesian coordinates in an XY plane) and whose thickness (in a Z direction) is relatively small (i.e., 1/10 or less) in comparison to its length (in an X direction) and width (in a Y direction). Non-limiting examples of substrates include a layer or layers of fibrous materials, nonwovens, films and foils such as polymeric films or metallic foils. These materials may be used alone or may comprise two or more sub-layers.

Within the present context, a "fibrous web" comprises a matrix of single type of fibers or filaments, or of a mixture, as may be directionally or randomly oriented, and bonded by friction, and/or adhesion, and/or cohesion, whereby the latter may be imparted directly after filament formation, e.g., at the lay-down step of fiber or filament forming when exhibiting a temperature above the non-tack temperature, or in a subsequent bonding step. Typically, a fibrous web is self-supporting and allows handling on manufacturing or converting equipment, though just a sub-web of a composite web may not have sufficient integrity alone and may be supported by a support equipment, such as a vacuum belt system and/or a support material, such as another web, as may be produced separately (off-line) and delivered as a roll good, or which may be formed in-line in an upstream (sub-)web-forming process step.

A "continuous web" is essentially endless in the length or x-direction corresponding during its manufacturing to machine direction (MD), as may be wound on rolls, or spools, or "festooned" in boxes, which may be spliced together to form the essentially endless web. Thus, a continuous web exhibits a width or y-direction perpendicular to the length or x-direction,

A web may comprise particulate additives, in particular additives which enhance absorbency of the composite web. The particular additive may include short cellulose-based fibers formed by a variety of pulping processes, such as kraft pulp, sulfite pulp, thermomechanical pulp, etc. The pulp fibers may include softwood fibers having an average fiber length of greater than 1 mm and particularly from about 2 to 5 mm based on a length-weighted average. Such softwood fibers can include, but are not limited to, northern softwood, southern softwood, redwood, red cedar, hemlock, pine (e.g., southern pines), spruce (e.g., black spruce), combinations thereof, and so forth. Hardwood fibers, such as eucalyptus, maple, birch, aspen, and so forth, can also be used. In certain instances, eucalyptus fibers may be particularly desired to increase the softness of the web. Eucalyptus fibers can also enhance the brightness, increase the opacity, and change the pore structure of the web to increase its wicking ability. Moreover, if desired, secondary fibers obtained from recycled materials may be used, such as fiber pulp from sources such as, for example, newsprint, reclaimed paperboard, and office waste. Further, other natural fibers can also be used in the present invention, such as abaca, sabai grass, milkweed floss, pineapple leaf, and so forth. In addition, in some instances, short synthetic fibers can also be utilized.

Absorbent webs may comprise superabsorbent polymers, preferably in particulate form, which are adapted to absorb liquids, such as water or aqueous solutions, such as bodily exudates, at multiples of their own weight. "Superabsorbent polymers" (SAP) refers herein to absorbent materials that can absorb at least 8 times their weight of an aqueous 0.9% saline solution as measured using the Centrifuge Retention Capacity (CRC) test (EDANA method NWSP 241.0.R2 (19)). The SAP preferably have a CRC value of at least 15 g/g. SAP are typically water-insoluble but water-swellable cross-linked polymers capable of absorbing large quantities of fluids. SAP are in particulate form so as to be flowable in the dry state. Typical particulate SAP are polyacrylate polymers, however it is not excluded that other polymer materials may also be used. For example, starch-based absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile. The particles may be relatively small (under 1 mm in their longest dimension) in their dry state and may be roughly circular in shape, but granules, fibers, flakes, spheres, powders, platelets and other shapes and forms are also known to persons skilled in the art.

The SAP may further be mixed with other dry additives, such as odour control material, antibacterial agents, such as without limitation active carbon, high surface area minerals, natural materials like herbs or herbal extracts, enzymatically active materials, or perfumes, all of which may be as such or be in a coated or micro-encapsulated form.

A particularly suitable application for such webs is in hygiene articles. As used herein, the term "absorbent articles" refers to devices that absorb and contain body exudates, and, more specifically, refers to devices that are placed against or in proximity to the lower torso of the wearer to absorb and contain the various exudates discharged from the body. Absorbent articles may include diapers, training pants, adult incontinence undergarments, feminine hygiene products, and the like. As used herein, the term "body fluids" or "body exudates" includes, but is not limited to, urine, blood, vaginal discharges, sweat and fecal matter.

Such absorbent articles are typically produced by a converting process operated on a converting line or machine, also referred to as "converter". On such a converter, continuous or composite webs are moved along a manufacturing path, as may define the machine direction, by drive systems, such as nip rolls, and further continuous webs or individual components, as may also be composed in sub-paths, are added thereto and connected by adhesive and/or thermal, pressure, or ultrasonic bonding. Thus, an absorbent article may be constructed by the combination of various components, such as backsheets, topsheets, ears, leg cuffs, elastic waist features, and absorbent cores, as may be added in separate or combined manufacturing units of the converter.

The term "machine direction" (MD) is used herein to refer to the direction of material flow through the manufacturing process. In addition, relative placement and movement of material can be described as flowing in the machine direction through a process from upstream in the process to downstream in the process along a manufacturing path, as may comprise sub-paths, such as when one web arriving on a first sub-path is combined with another web or structure arriving on a second sub-path, and both being combined to further travel along the combined path. The term "cross direction" (CD) is used herein to refer to a direction that is generally perpendicular to the machine direction.

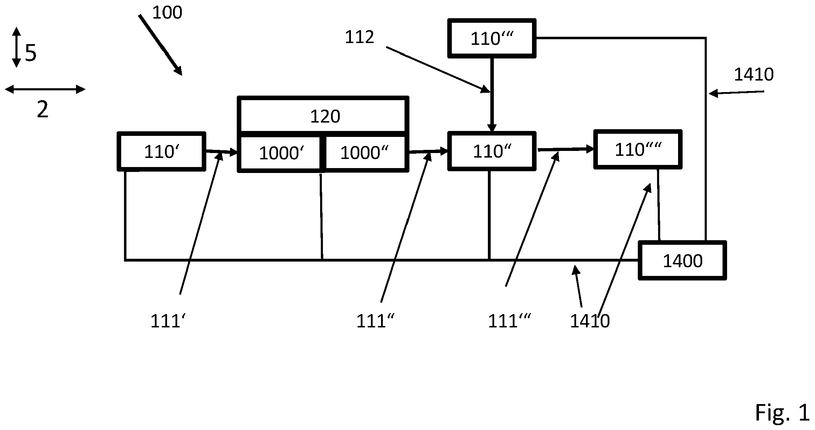

Referring for explanatory but not limiting purposes to Fig 1, such a converter 100 for the manufacturing of a hygiene article comprises multiple converting units 110', 110", ... as may be arranged along the machine direction 2 (MD) along manufacturing path segments 111', 111", ... or side path segments 112 connecting the various converting units. Manufacturing unit 110"" may exemplarily depict the end of the manufacturing process, e.g., a packaging or even palletizing unit. Within the present context, MD of a converter or converting units therein is generally aligned with the longitudinal direction of the structures or web(s), and CD (8) with the width direction thereof, although process variants may exist, where the structure is turned by 90° before being combined with other elements to form the absorbent article. In a plane section of the equipment, the thickness of the structures or web(s) corresponds to the height of the unit oriented along gravity. However, a path of a web or structure may bend or turn, typically upwardly or downwardly along gravity, and the thickness-direction of the web or structure may change accordingly relative to gravity, but not relative to the width/length orientation of the structure and the process. Referring explanatorily to Fig. 1, the main manufacturing direction is shown horizontally along manufacturing path segments 111',1111", and 111‴. However, for sub-path 112 from manufacturing unit 110'" to unit 110" the manufacturing sub-path machine direction may be vertical.

In the context of hygiene or absorbent articles, "longitudinal" means a direction running substantially from the front waist portion of a wearer through the crotch region to the rear waist region in a generally U-shaped in-use configuration. In the manufacturing configuration, the structure or the article may be essentially flat or folded. Directions within 45 degrees of the longitudinal direction are considered to be "longitudinal." "Lateral" or widthwise refers to a direction running from a longitudinally extending side edge to a laterally opposing longitudinally extending side edge of an article - generally at a right angle to the longitudinal direction - and corresponds to a left-right orientation in the in-use configuration on a wearer. Directions within 45 degrees of the lateral direction are considered to be "lateral."

In modern production units, the main manufacturing path 111 typically corresponds to a single continuous web or two or more webs forming am essentially continuous composite web along the main manufacturing path, to which other components are added.

If in any of the manufacturing units an error or failure occurs, for example the tearing of a web or a misalignment of webs that cannot be automatically corrected, or the plugging of hot-melt glue applicator, the complete process comes to a halt, based upon a signal from various control elements, such as optical sensors to detect off-positioning of elements, typically controlled via a central control unit 1400 connected to the various process units via process control connections 1410. This results in the generation of scrap for any stop causing off-target products, but also for the transition periods, i.e., ramp-down and ramp-up, wherein the scrap includes all elements already added to the process at that point in time.

When hot-melt glues are applied, also his application is stopped and the molten adhesive remains in the nozzle head in an "idle" state of operation. However, for such materials it is not so critical as maintaining it at the melting temperature is not causing major degradation of the polymer and the respective properties.

It is an important aspect of the present invention that an apparatus according to the present invention 1000 may be included into such a converting line, optionally also multiple apparatuses, as may also be aggregated in a composite web forming unit 120, but the filament forming is set to an "idle" or non-production state of operation during which the filament forming is not interrupted but the formed filaments are separately collected for recycling, which can be fed into the filament forming process or to other uses of the polymer which remains essentially clean and undeteriorated.

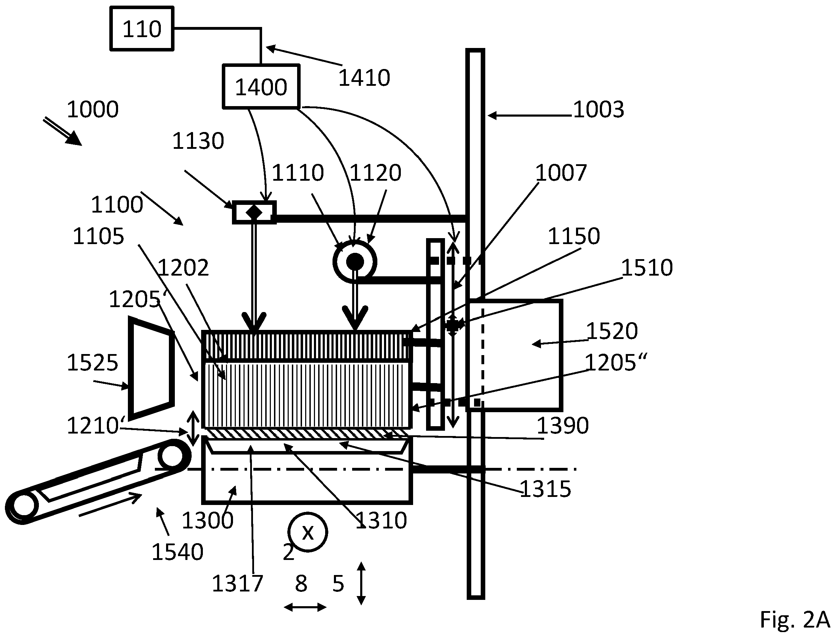

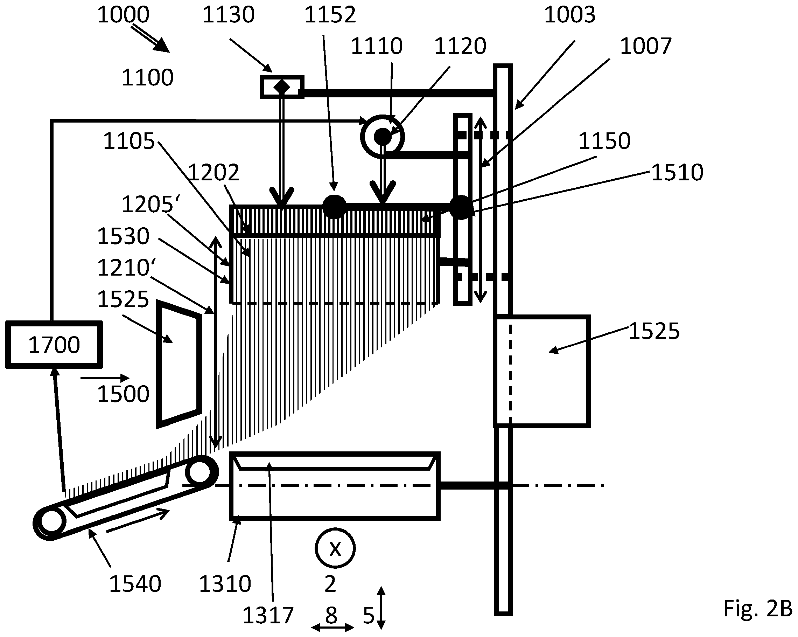

To this end, an apparatus 1000 according to the present invention is now explained by referring to Fig. 2A and B, which however should not be seen as limiting the executions as described in the following.

The apparatus comprises the well-known systems for forming webs with continuous filaments:
a) A fixed frame 1003 adapted to connect systems of the apparatus stationarily or movably thereto, whereby walls or floors can be part of the frame construction.
b) A primary filament collecting system 1300 fixedly connected relative to the frame and comprising at least one primary collection surface 1315 adapted to form thereon a web of filaments and transferring the web towards a downstream processing unit.

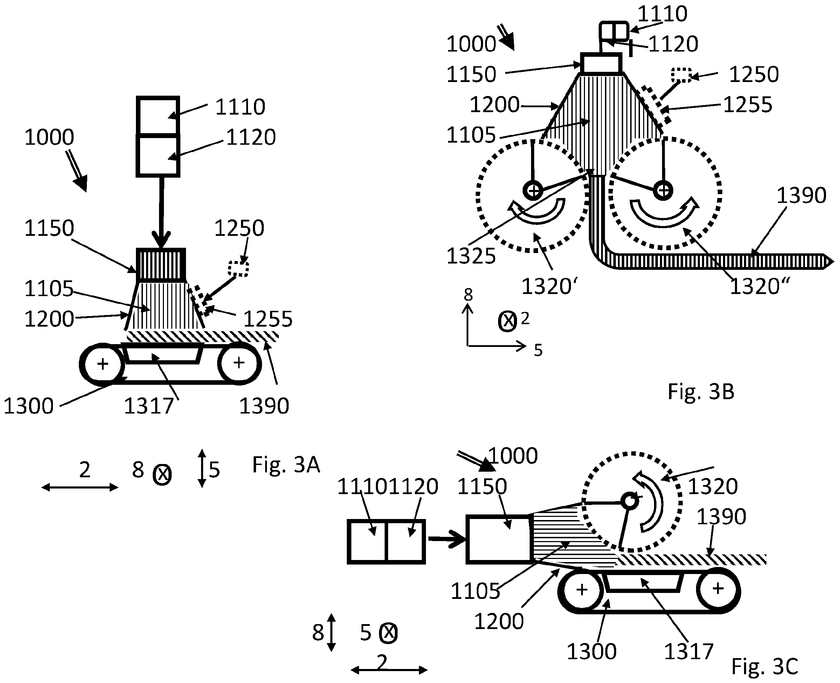

In one execution as indicated in Fig. 2A and 3A, the primary collection system 1300 may be a foraminous belt system 1310 with a suction box 1317, as well known in the art.

In another execution, as may be preferred when webs with hight loft or lower density are desired, the primary collection system 1300 may be a system of two counter-rotating drums 1320 where the filaments 1105 are expelled towards a gap 1325 of typically from more than about 0.5 mm to less than about 100 mm between the drums where these accumulate to a web of predetermined thickness, see Fig. 3B. As shown in Fig. 2A, the manufacturing direction of the web forming in the gap 1325 can be seen along the vertical 8, thought the formed web 1390 may then be diverted into the horizontal 5.

Whilst for the first two executions the filament forming may be along the vertical direction 5, a third execution for the primary collection system 1300 may be the combination of a foraminous belt 1300 and one drum 1320 with the filaments 1105 being blown into a gap formed between the two, see Fig. 3C, which, however, may increase complexity within the present context, as will described herein below.
c) Referring again to Fig. 2A, the apparatus 1000 further comprises an essentially continuously operating, continuous filament forming system 1100 preferably of the spunbonding or even more preferably of the coaxial meltblowing type,
   comprising
   c1) a polymer supply (1110) and extruding (1120) unit adapted to melt polymer;
   c2) a molten polymer filament expelling unit (die head) 1150;
   c3) a filament quenching and attenuation system 1130;
d) Often, and essential for the present application, the unit further comprises a housing 1200 circumscribing a space between the filament expelling unit 1150 and the primary filament collecting device 1320, wherein the housing comprises
   d1) a first opening 1202 to receive the filament expelling unit and a second opening 1208 towards the primary collection unit;
e) control and adjustment system (1400 - only shown in Fig. 2A and omitted in Fig. 2B) for adapting process conditions of the apparatus 1000, the process conditions comprising at least
   e1) a polymer temperature at the filament expelling unit 1150,
   e2) a polymer throughput through the filament expelling unit 1150,
   e3) a quenching air flow & temperature regulating unit;
   wherein the control and adjustment system 1400 is adapted to receive a signal via process control signal connection 1410 from the further processing unit(s) 110 indicating regular production state or non-production state, e.g., idle or stop or scrap).

In order to adapt to the interruption of the manufacturing process, an apparatus 1000 according to the present invention allows to divert the continuously produced filaments 1105 of the filament forming system 1100 away from the primary filament collection system 1300 of the main production towards a secondary filament collection system 1500, where the filaments of the essentially undeteriorated polymer are collected on a secondary filament transfer system (1540) and may be processed into the polymer extruding unit 1120, preferably stored for further processing, e.g., via a filament recycling system 1700, e.g., a re-granulating unit, or to other polymer using units.

To this end, in an apparatus 1000 according to the present invention the filament forming system 1100 and / or at least the filament expelling unit 1150 are moveably connected via a moveable frame 1007 to the frame 1003, such that the distance 1210 between the filament expelling unit 1150 and the primary filament collection unit surface 1315 can be adjusted by a distance adjustment system 1510, i.e., to be increased from a first distance 1210' (Fig. 2A) for the state of regular production to a second distance 1210" (Fig. 2B) for the state of interrupted production and "idle" for the filament formation.

The housing 1200 may also be moved together with the filament expelling unit 1150 or by a separate housing dislocation system 1530 and concurrently or alternatively lateral portions 1205 thereof may be moved laterally away from their "production" position, thereby opening the space for a cross-directional cooling and displacement gas flow induced by a blower (1525) and suction (1520) system positioned cross-directionally opposed to each other and dislocating the filaments away from the primary collection system 1300 towards a secondary filament transfer system 1540. As exemplarily depicted in the figure, the secondary transfer system 1540 may be a foraminous belt system or just a collection baffle. As the filaments are not sticky anymore, preferably due to the cooling of the blower / suction effect with cooling air from the suction / blower system 1525/1520, these may be readily handled and further transferred to filament recycling system 1700 (see Fig. 2B only), e.g., an intermediate storage bin, and/or a re-granulator and/or directly to the polymer extruding unit.

In addition, the control unit allows monitoring other processing parameter, in particular any failures or defects that require stopping of the regular production process and toggling between a regular production state and a non-production state. For the latter, other process units such as unwinding unit for webs or glue applicators are stopped, and during the transition between the states the produced articles or precursors thereof are scrapped, optionally being transferred to a factory scrap recycling unit, where portions of the materials may be reclaimed, albeit typically with a deterioration of materials properties, mostly due to contamination with other materials.

For the present invention, the "stop regular production" signal puts the filament forming apparatus 1100 into a non-production state, by increasing the distance of the filament expelling unit 1150 and the primary filament collection system, preferably jointly with the fixedly connected polymer extruding unit 1120 and activating the blower / suction system 1525 / 1520 and the secondary transfer system 1540.

Preferably, concurrently the process conditions of the apparatus are adjusted to a set of non-production state conditions, typically with lower energy requirements without losing the capability of a quick re-start, at least for
▪ a polymer temperature at the filament expelling unit 1150,
▪ a polymer throughput through the filament expelling unit 1150,
▪ a quenching air flow & temperature regulating unit 1130.

Such toggling between a regular production state and a non-production state can happed quite quickly, and may be less than about 10 second, preferably less than about 5 seconds and more preferably even less than about 3 seconds, thereby creating only a small amount of transition scrap. Once the cause for the interruption of the main production process is eliminated, the central control system 1400 provides a signal for starting up the other systems but also the filament web forming system by resetting the process conditions, stopping the air blowing / suction system 1525 / 1520 and the secondary filament collection system 1400 and repositioning the movable elements.

In an optional execution of the present invention, the polymer filament expelling unit 1150 may be rotatably mounted around an axis parallel to the direction of the expelled filaments 1105, i.e., a vertical axis 1152 in the configuration as depicted in Fig. 2B. Such an arrangement allows to adjust the width of the web as deposited on the primary collection system 1300. This allows very flexible size changes in the articles as produced on the line without the need for replacing material supply, such as rolls, or even size change parts.

In a preferred execution, the housing opening and relocation system 1530 comprises a set of at least two shells positioned cross-directionally opposite and adapted to be at least cross-directionally spaced apart.

The filaments as collected by the secondary filament collection system 1500 may be refed to the polymer supply 1110 for the filaments. This is possible, as the polymer is essentially not deteriorated during the filament forming and collection due to the short residence times in this system and to the avoidance of too high temperatures. Depending on the exact execution of the polymer handling system, the filaments may be chopped and fed into the polymer supply. Often it may be preferred to granulate the filaments and add these, optionally with an interim storage. Alternatively, the polymer may be provided to other uses.

The housing 1200 may be adapted to receive additives to be intermingled with the filaments during the production state and provided by an additive supply 1250 through an additive infeed system 1255 (both indicated in dashed lines in Fig. 3A and B). As described in the above, such additives may be - without limitation - particulate additives, such as the above-mentioned short cellulose based fibers, or particles, such as the above-mentioned SAP particle, thereby creating absorbent structures. During the non-production state, such additives can be quickly shut off, thereby leaving the filaments collected by the secondary collection system uncontaminated for further recycling or refeeding.

Optionally, for certain applications it might be beneficial to use a preformed web, such as a high loft fibrous web, and to deposit the filaments - pure or together with an additive - onto such a web, whereby at least some of the additives and / or filaments may penetrate into the pores of such an open web.

A particular execution of the present invention relates to a composite web forming equipment comprising two or more apparatuses as described in the above, adapted to toggle between a production state and a non-production state by a common control system receiving the signal.

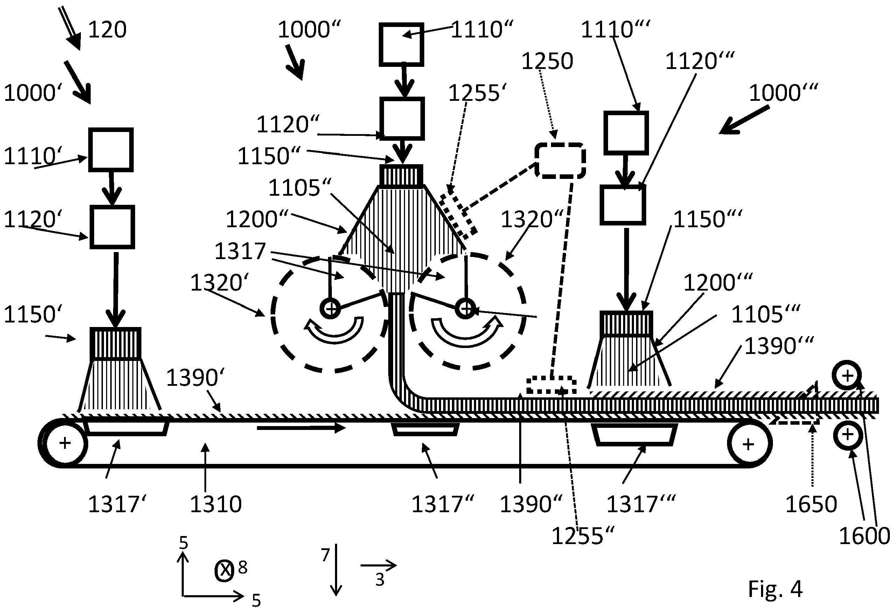

A particularly preferred exemplary composite web forming equipment 120 is depicted in Fig. 4, as may be suitably used for producing an absorbent structure. To this end, a first filament forming system 1000' deposits a first scrim layer 1390', preferably of low basis weight, onto a first deposition region with a first suction box 1317' of a collection system, here exemplarily shown as a common filament collection system 1310. In a second filament forming system 1000", SAP particles from a particulate material supply system 1250 are intermingled via the particulate material infeed 1255' with continuous filaments 1105". This particle / filament intermix may be deposited onto a system of counterrotating foraminous drums 1320', 1320", thereby adjusting the density of the intermix, which may then be deposited in the region of a second suction box 1317" onto the first scrim layer 1390', forming an intermediate composite web 1390". Additionally or alternatively, the particulate additive may be added after the filaments are deposited onto the filamentary web, indicated by a particulate additive system 1255".

Then in a third filament forming system 1000'", a second scrim layer may be positioned onto of the intermediate composite web 1390". As the scrim layers may exhibit somewhat irregular lateral edges, these may be straightened in an edge straightening system 1650 according to the predetermined width of the final composite web 1390'", such as by removing protruding portions and recycling these, or by overfolding these onto the web, thereby even further increasing the containment of particulate material, if present.. A conventional bonding unit 1600, preferably an ultrasonic bonding unit such as known from e.g., WO2014/001487A1 (C4S), completes the formation of an absorbent structure, as may suitably be fed into the further production of an absorbent article, such as by a conventional "cut and place" unit for spacing subsequent absorbent structures. All three filament forming systems 1000 can be movably connected to the frame and be repositioned together upon interruption of the process, and also all three units may be controlled by a common control unit. As will be appreciated by a skilled person, it is more complex to combine a filament forming system with a gap formed between a foraminous drum and belt, as depicted in Fig. 3C, with vertical systems as depicted in Fig. 3A and 3B, as the distance adjustment system needs to increase the distance in the vertical direction for the vertical system, but in the horizontal for the horizontal system. It should be noted that the polymers for the three filament forming systems may be identical and even be fed from a single extruder, but for certain executions it may be preferable that these are different, e.g., providing more resilient polymers, such as polyester, to the center unit and softer polymers, such a polyolefins, e.g., polypropylene, to the scrim layers. The center unit may also be provided with more elastic polymers, such as the above referenced polypropylene / propylene/α-olefin copolymer blends.

## Claims

1. An apparatus (1000) for the production of a web (1390) comprising continuous filaments (1105), said apparatus (1000) exhibiting a machine direction MD (2), a cross-machine direction CD (8), and a height or z (5) -direction and
being adapted to be connected MD directionally upstream and/or downstream to further processing units (110) for forming composite structures comprising said web;
said apparatus (1000) comprising
a) a frame (1003) adapted to connect systems of said apparatus stationarily or movably thereto;
b) a primary filament collecting system (1300) connected to said frame (1003) directly or indirectly, and comprising at least one primary collection surface (1315) adapted to form thereon a web of filaments and transferring said web towards a downstream processing unit;
c) an essentially continuously operating filament forming system (1100) comprising
c1) a polymer supply (1110) and extruding (1120) unit adapted to melt polymer;
c2) a molten polymer filament expelling unit (1150) being movably connected to said frame so as to allow repositioning relative to said primary collection system (1300);
c3) a filament quenching and attenuation system (1130);
d) a housing (1200) circumscribing a space between said filament forming device (1100) and said primary filament collecting device (1300), said housing (1200) comprising
d1) a first opening (1202) to receive said filaments (1105) from said filament expelling unit (1150) and a second opening (1208) towards said primary collection unit (1300);
d2) movable cross-directionally oppositely positioned portions (1205) adapted to open said space at least laterally;
e) a control and adjustment system (1400) for adapting process conditions of said apparatus (1000),
said process conditions comprising at least
∘ a polymer temperature at said filament expelling unit (1150),
∘ a polymer throughput through said filament expelling unit (1150),
∘ a quenching air flow & temperature regulating unit (1130);
wherein said control and adjustment system (1400) is adapted to receive a signal from said further processing unit(s) (110) indicating regular production state or non-production state;
wherein
e) said apparatus (1000) further comprises a secondary filament collection system (1500) comprising
- e1) a distance adjustment system (1510) to adjust the distance between said filament expelling device (1150) and said primary filament collecting surface (1315) to a first production state distance (1210') or a second non-production state distance (1210");
- e2) a cross-directional gas flow system comprising a blower (1525) and suction (1520) unit for creating a cross-directional air flow between said primary collection surface (1315) and said filament expelling unit (1150) during said non-production state,
- e3) a housing relocation system (1530) adapted to move said cross-directionally oppositely positioned portions (1205) of said housing so as to allowing said cross-directional airflow to contact said filaments (1105) and transferring these cross-directionally during said non-production state;
- e4) a secondary filament transfer system (1540) adapted to receive said cross-directionally transferred filaments and to put these into a storage system;
wherein said secondary filament collection system (1500) is operated upon said signal from said control and adjustment system (1400) toggling between regular production and non-production state.

2. An apparatus according to claim 1, comprising further one or more of the features selected from the group consisting of:
- said continuously operating filament forming system (1100) is selected from the group consisting of spunbonding and coaxial meltblowing, preferably coaxial meltblowing;
- said primary filament collecting device (1300) is selected from the group consisting of
∘ a foraminous belt system (1310);
∘ a double foraminous drum system (1320', 1320");
∘ a foraminous belt and drum system (1310, 1320);
- said cross-directional gas flow system is adapted to cool said filaments below a polymer stickiness temperature;
- said polymer extruding unit (1120) and said filament expelling unit (1150) are connected and jointly moved by said distance adjustment system (1510);
- said polymer filament expelling unit (1150) is rotatably mounted with an axis (1152) parallel to the direction of the expelled filaments;
- said housing opening and relocation system (1530) comprises a set of at least two shells positioned cross-directionally opposite and adapted to be at least cross-directionally spaced apart.

3. An apparatus according to claim 1 or 2,
further comprising a filament recycling system (1700) of said filaments from said secondary filament transfer system (1540) of said secondary filament collection system (1500), preferably comprising a polymer granulator and a mixer for feeding fresh and recycled polymer to said polymer extruding unit (1120).

4. An apparatus according to any of the preceding claims,
further comprising a particulate supply (1250) and feeding (1255) system, preferably through further openings of said housing (1200), adapted to feed particulate additives to be intermingled with said filaments (1105) during said production state but not during said non-production state.

5. An apparatus according to any of the preceding claims, positioned downstream of a preformed web supply unit for positioning a preformed web between said primary filament collection unit (1300) and web of filaments formed thereon.

6. A composite web forming equipment (120) comprising two or more apparatuses (1000) according to any of claims 1 to 5, adapted to toggle between a production state to a non-production state by a common control system (1400) receiving a signal from further production units (110).

7. A process for the production of a web of continuous filaments,
said process being connected machine-directionally (MD) upstream and/or downstream to further processing steps on further processing units (110) for forming composite structures comprising said web,
said process being operated along a main machine direction and further exhibiting a cross-direction (CD) and a z- or height direction, rectangularly to MD and CD;
said process comprising the steps of
A) providing
- an apparatus or equipment as per any of claims 1 to 7;
- polymer for forming filaments;
B) operating said one or more essentially continuously operating continuous filament forming system(s) (1100) at regular production process conditions of at least
∘ a polymer temperature at said filament expelling unit (1150),
∘ a polymer throughput through said filament expelling unit (1150),
∘ a quenching air flow & temperature at a quenching air flow & temperature regulating unit (1130);
C) interrupting said step B) upon receipt of a signal from a further processing unit(s) (110), preferably via a central control unit (1400) indicating non-production state,
thereby essentially concurrently
C1) adjusting said process conditions of said filament forming system (1100) according to pre-set non-production process conditions;
C2) activating said secondary filament collection system (1500) by
C2i) initiating said housing opening and relocation system (1530) so as to
∘ increase the distance (1210) between said filament expelling device (1150) and said primary filament collecting surface (1300), and
∘ opening movable cross-directionally oppositely positioned portions (1205) of said housing (1200) so as to open said space at least laterally;
C2ii) initiating said cross-directional gas flow system (1520/1525) so as blow said filaments laterally towards said secondary filament transfer system (1540), thereby preferably reducing the temperature of said filaments below a non-tack temperature;
C2iii) initiating said secondary filament transfer system (1540), preferably transferring the filaments collected thereon to filament recycling system (1700),
whereby said steps B) and C) are alternatingly operated upon receipt of said signal from a further processing unit (110) or said central control unit (1400).

8. A process according to claim 7, wherein further
- the transition time between the production state (step B) and the non-production state (step C) is
∘ less than 10 sec, preferably less than 5 sec, more preferably less than 1 sec;
∘ more than about 10 msec;
- the polymers are selected from the group consisting of
∘ polyolefins, preferably polypropylene, polypropylene copolymers, polyethylene, polyethylene copolymers, polyesters, polyamides, polyimide, polylactic acid, polyhydroxyalkanoate, polyvinyl alcohol, ethylene vinyl alcohol, nylon, polyacrylates, and copolymers thereof and mixtures thereof.
